# EUROPEAN PATENT SPECIFICATION

(11) **EP 1 931 711 B1**
(45) Date of publication and mention of the grant of the patent: **08.04.2009**
(21) Application number: 06805884.1
(22) Date of filing: 27.09.2006
(51) Int. Cl.: C07K 16/26

(54) **ANTIBODIES AGAINST 25-HYDROXYVITAMIN D**
ANTIKÖRPER GEGEN 25-HYDROXYVITAMIN D
ANTICORPS AGISSANT CONTRE LA 25-HYDROXYVITAMINE D

(30) Priority: 29.09.2005 EP 05021247
(43) Date of publication of application: 18.06.2008
(73) Proprietor: Roche Diagnostics GmbH, 68305 Mannheim (DE); F.HOFFMANN-LA ROCHE AG, 4070 Basel (CH)
(72) Inventor: HUBER, Erasmus, 86923 Finning (DE); BECKER, Juergen, 82377 Penzberg (DE); KRAUS, Werner, 82362 Weilheim (DE); KYRIATSOULIS, Apostolos, 82362 Weilheim (DE); VOGEL, Rudolf, 82362 Weilheim (DE); HORN, Nicole, 82343 Niederpoecking (DE)
(86) International application number: PCT/EP2006/009360
(87) International publication number: WO 2007/039193

(56) References cited:
- EP-A2- 0 312 360
- MCVICAR J P ET AL: "CHARACTERISTICS OF HUMAN LIPO PROTEINS ISOLATED BY SELECTED AFFINITY IMMUNO SORPTION OF APO LIPO PROTEIN A-I/" PROCEEDINGS OF THE NATIONAL ACADEMY OF SCIENCES OF THE UNITED STATES OF AMERICA, vol. 81, no. 5, 1984, pages 1356-1360, XP002415292 ISSN: 0027-8424
- KOBAYASHI NORIHIRO ET AL: "Production and specificity of antisera raised against 25-hydroxyvitamin D-3-(C-3-)-bovine serum albumin conjugates" STEROIDS, vol. 57, no. 10, 1992, pages 488-493, XP002415293 ISSN: 0039-128X
- HUMMER L ET AL: "A SELECTIVE AND SIMPLIFIED RADIOIMMUNOASSAY OF 25-HYDROXYVITAMIN D3" SCANDINAVIAN JOURNAL OF CLINICAL AND LABORATORY INVESTIGATION, BLACKWELL SCIENTIFIC PUBLICATIONS, OXFORD, GB, vol. 44, no. 2, 1984, pages 163-167, XP009017542 ISSN: 0085-591X
- KOBAYASHI NORIHIRO ET AL: "PRODUCTION AND CHARACTERIZATION OF MONOCLONAL ANTIBODIES AGAINST TWO HAPTENIC DERIVATIVES OF 1ALPHA,25-DIHYDROXYVITAMIN D3 CONJUGATED WITH BOVINE SERUM ALBUMIN THROUGH THE C-3 OR C-24 POSITION" BIOLOGICAL & PHARMACEUTICAL BULLETIN (OF JAPAN), PHARMACEUTICAL SOCIETY OF JAPAN, TOKYO, JP, vol. 20, no. 9, 1997, pages 948-953, XP001248794 ISSN: 0918-6158
- KOBAYASHI N ET AL: "Production and characterization of monoclonal antibodies against a novel 1alpha,25-dihydroxyvitamin D3-bovine serum albumin conjugate linked through the 11alpha-position" JOURNAL OF STEROID BIOCHEMISTRY AND MOLECULAR BIOLOGY, ELSEVIER SCIENCE LTD., OXFORD, GB, vol. 63, no. 1-3, September 1997 (1997-09), pages 127-137, XP002272138 ISSN: 0960-0760
- HARRIS J R ET AL: "Keyhole limpet hemocyanin (KLH): A biomedical review" MICRON, vol. 30, no. 6, December 1999 (1999-12), pages 597-623, XP002415294 ISSN: 0968-4328

## Description

### Background information

The present invention concerns processes for the production of antibodies against 25-hydroxyvitamin D, the antibodies produced according to the invention as well as methods for detecting 25-hydroxyvitamin D using these antibodies.

An adequate supply of vitamin D is vital as the term "vitamin" already suggests. A deficiency of vitamin D leads to severe diseases such as rickets or osteoporosis. While vitamin D was still regarded as a single substance at the beginning of the last century, the vitamin D system has developed further in the course of the last three decades into a complex and manifold network of vitamin D metabolites. Nowadays more than 40 different vitamin D metabolic products are known (Zerwekh, J.E., Ann. Clin. Biochem. 41 (2004) 272-281).

Humans can only produce D₃ vitamins or calciferols by the action of ultraviolet rays from sunlight on the skin. Vitamin D₃ that is produced in the skin binds to the so-called vitamin D-binding protein which transports it into the liver where it is converted into 25-hydroxyvitamin D₃ by 25-hydroxylation. A multitude of other tissues are nowadays known to be involved in vitamin D metabolism in addition to the skin and liver, the two organs that have already been mentioned (refer to Schmidt-Gayk, H. et al. (eds.), "Calcium regulating hormones, vitamin D metabolites and cyclic AMP", Springer Verlag, Heidelberg (1990), pp. 24-47). 25-Hydroxyvitamin D and more specifically 25-hydroxyvitamin D₂ and 25-hydroxyvitamin D₃ are the central storage forms of vitamin-D in the human organism with regard to their amounts. When needed these precursors can be converted in the kidneys to form the biologically active 1α,25-dihydroacyvitamin D, the so-called D hormone. The biologically active vitamin D regulates among others calcium uptake from the intestine, bone mineralization and it influences a large number of other metabolic pathways such as e.g. the insulin system.

Measuring the vitamin D level itself is of little benefit when determining the vitamin D status of a patient, because concentrations of vitamin D (vitamin D₂ and vitamin D₃) fluctuate greatly depending on food uptake. In addition vitamin D has a relatively short biological half-life in the circulation (24 hours) and it is therefore also for this reason not a suitable parameter for determining the vitamin D status of a patient. The same also applies to physiologically active forms of vitamin D (1,25-dihydroxyvitamin D). These biologically active forms also occur in relatively small and highly fluctuating concentrations compared to 25-hydroxyvitamin D. For all these reasons the quantification of 25-hydroxyvitamin D in particular is a suitable means to globally analyse the total vitamin D status of a patient.

Due to the high clinical importance of 25-hydroxyvitamin D, a large number of methods are known from the literature which allow 25-hydroxyvitamin D to be more or less reliably determined.

Haddad, J.G. et al., J. Clin. Endocrinol. Metab. 33 (1971) 992-995 and Eisman, J.A. et al., Anal. Biochem. 80 (1977) 298 - 305 for example describe the determination of 25-hydroxyvitamin D concentrations in blood samples using high performance liquid chromatography (HPLC).

Other approaches for the determination of 25-hydroxyvitamin D are based among others on the use of vitamin D binding proteins like those that are present in milk. Thus Holick, M.F. and Ray, R. (US 5,981,779) and DeLuca et al. (EP 0 583 945) describe vitamin D assays for hydroxyvitamin D and dihydroxyvitamin D which are based on the binding of these substances to vitamin D-binding protein where the concentrations of these substances are determined by means of a competitive test procedure. However, a prerequisite of this method is that vitamin D metabolites to be determined firstly have to be isolated from the original blood or serum samples by organic extraction and have to be purified by, for example, chromatography.

Armbruster, F.P. et al. (WO 99/67211) teach that a serum or plasma sample should be prepared for vitamin D determination by ethanol precipitation. In this method the protein precipitate is removed by centrifugation and the ethanolic supernatant contains soluble vitamin D metabolites. These can be measured in a competitive binding assay.

Alternatively EP 0 753 743 teaches that the proteins can be separated from blood or serum samples using a periodate salt. In this case vitamin D compounds are determined in the protein-free supernatant from the samples treated with periodate. In some commercial tests acetonitrile is recommended for the extraction of serum or plasma samples (e.g. in the radioimmunoassay from DiaSorin or in the vitamin D test from the "Immundiagnostik" Company).

In recent years a number of different release reagents were proposed which should in principle be suitable for releasing vitamin D compounds from binding protein present in the sample. However, this release or detachment should be carried out under relatively mild conditions thus enabling a direct use of the sample treated with the release reagent in a binding test (see for example WO 02/57797 and US 2004/0132104). Despite immense efforts in recent years, all available methods for determining vitamin D have certain disadvantages such as laborious sample preparation, poor standardization, poor agreement between test procedures or bad recovery of spiked vitamin D (see for this in particular Zerwekh, J.E., supra).

EP 03 120 360 and Kobayashi et al (Steroids, 1992, vol. 57, pages 488-493) disclose the preparation of 25-hydroxy vitamin D3 derivatives bearing a hapten-carrier in position 3.

Hummer et al (Scand. J. clin. Lab. Invest., 1984, vol. 44, pages 163-167) describes a method for determining 25OHD3 in serum by adding vitamin D2 in the sample.

In particular no methods are described in the prior art that can be used to reliably produce antibodies for determining 25-hydroxyvitamin D. The object of the present invention was therefore, among others, to find a method which can be used to reliably produce suitable antibodies for a 25-hydroxyvitamin D test. Such a method, the antibodies produced by the method, as well as methods and kits for determining vitamin D using these antibodies are described in the following.

### Summary of the invention

The present invention concerns a process for producing antibodies against 25-hydroxyvitamin D which comprises the following steps:
a) immunizing an experimental animal with a conjugate which contains 25-hydroxyvitamin D₃ or 25-hydroxyvitamin D₂ as the hapten,
b) isolating serum or plasma from the said experimental animal and
c) purifying the antibodies contained in the serum or plasma by immunosorption.to a complementary matrix, comprising 25-hydroxyvitamin D₂ or 25-hydroxyvitamin D₃, respectively.

Furthermore the invention concerns antibodies against 25-hydroxyvitamin D₃ which have a cross-reaction with 25-hydroxyvitamin D₂ of the order of magnitude of 10 % to 1000%.

The present application also describes how the antibodies according to the present invention can be used for an automated test to detect 25-hydroxyvitamin D.

In addition a test kit for detecting 25-hydroxyvitamin D is disclosed which contains the reagent compositions required for the test procedure and among others the antibodies against 25-hydroxyvitamin D according to the invention.

The present invention concerns a process for producing antibodies against 25-hydroxyvitamin D which comprises the following steps:
a) immunizing an experimental animal with a conjugate which contains 25-hydroxyvitamin D₃ or 25-hydroxyvitamin D₂ as the hapten,
b) isolating serum or plasma from the said experimental animal and
c) purifying the antibodies contained in the serum or plasma by immunosorption to a complementary matrix, comprising 25-hydroxyvitamin D₂ or 25-hydroxyvitamin D₃, respectively.

If not stated otherwise the term "vitamin D" is understood to include the forms of vitamin D₂ and vitamin D₃ according to the following structural formulae I and II.

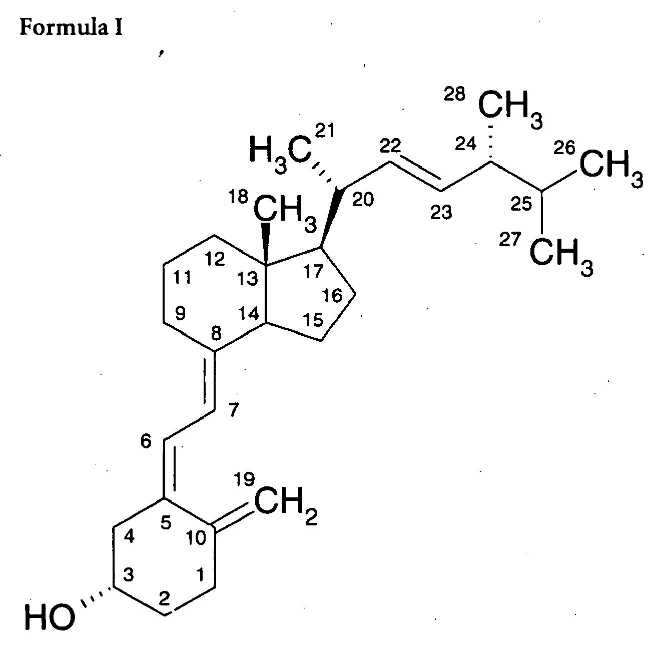

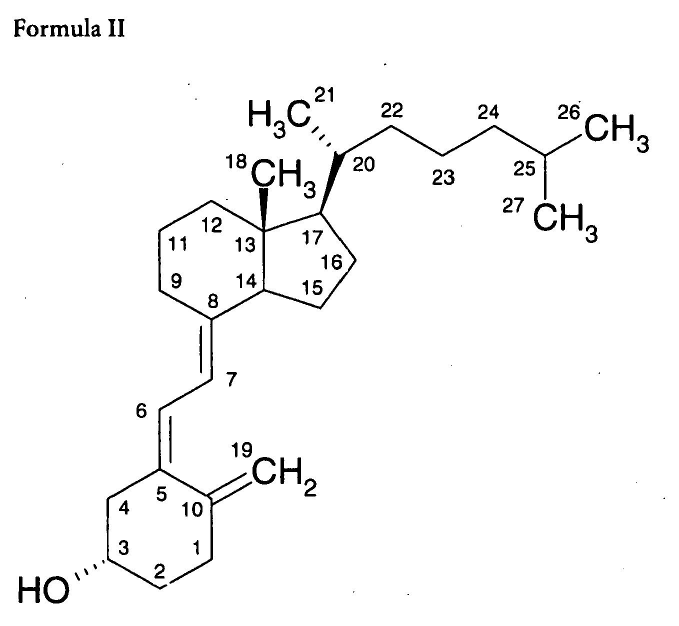

In the structural formulae I and II the positions of vitamin D are stated according to the steroid nomenclature. The 25-hydroxyvitamin D denotes vitamin D metabolites that are hydroxylated at position 25 of the structural formulae I and II i.e. 25-hydroxyvitamin D₂ as well as 25-hydroxyvitamin D₃. As already elucidated above, 25-hydroxyvitamin D₂ and 25-hydroxyvitamin D₃ are particularly relevant forms of vitamin D for diagnostics.

1,25-Dihydroxyvitamin D refers to the active forms of vitamin D (the so-called D hormones) that have a hydroxylation at position 1 as well as at position 25 of the structural formulae I and II.

Other known vitamin D metabolites are 24-dihydroxyvitamin D₂ and 25-dihydroxyvitamin D₂ as well as 24-dihydroxyvitamin D₃ and 25-dihydroxyvitamin D₃.

All known vitamin D metabolites are as such not immunogenic. The chemical activation of components from vitamin D metabolism as well as their coupling to carrier molecules or reporter groups is not trivial. Thus for a successful immunization it is essential to prepare a conjugate which for example contains a 25-hydroxyvitamin D as a hapten. The term hapten is understood by a person skilled in the art as a substance which per se is not immunogenic but, by coupling to a larger carrier molecule, is present in a form against which antibodies can be generated. Suitable carrier materials for the production of hapten conjugates are known to a person skilled in the art. Bovine serum albumin, β-galactosidase or the so-called keyhole limpet hemocyanine (KLH) are usually used as carrier materials.

KLH has proven to be a particularly suitable carrier for the method according to the invention. Hence a conjugate of 25-hydroxyvitamin D and KLH is preferably used for the immunization.

Various positions of the structures as they are shown in formula I and II are in : principle suitable for activation and coupling to a carrier material. Coupling via position 3 of 25-hydroxyvitamin D₂ or 25-hydroxyvitamin D₃ has for example proven to be favourable for the generation of antibodies which bind a 25-hydroxyvitamin D in a suitable manner. Hence in a preferred embodiment a conjugate is used in an immunization method according to the invention which contains 25-hydroxyvitamin D₃ or 25-hydroxyvitamin D₂ that has been coupled via position 3 of the backbone (cf. formulae I and II).

In a series of experiments that were part of the work for the present invention, attempts were made to purify antibodies that had been produced using a 25-hydroxyvitamin D₃ immunogen by immunosorption to a 25-hydroxyvitamin D₃ matrix and to use them in a corresponding test. However, these experiments were unsuccessful. However, it was surprisingly found that suitable antibodies can be obtained from the same sera by immunosorption to a 25-hydroxyvitamin D₂ matrix. This method has proven to be reliable and reproducible. The method according to the invention therefore comprises a step for purifying antibodies against 25-hydroxyvitamin Dₓ (where x =. 2 or 3) from serum or plasma by immunosorption to a matrix which contains a conjugate of the respective complementary form of the 25-hydroxyvitamin D. In this sense 25-hydroxyvitamin D₃ is complementary to 25-hydroxyvitamin D₂ and conversely 25-hydroxyvitamin D₂ is complementary to 25-hydroxyvitamin D₃. This means that immunosorption to 25-hydroxyvitamin D₂ is carried out when immunizing with 25-hydroxyvitamin D₃ and immunosorption to 25-hydroxyvitamin D₃ is carried out when immunizing with 25-hydroxyvitamin D₂.

Moreover, it has proven to be advantageous to use the same position of the vitamin D backbone for chemical coupling in the 25-hydroxyvitamin D conjugate used for the immunization and in the matrix used for the immunosorption. The coupling in the 25-hydroxyvitamin D₃ conjugate is preferably via position 3 of 25-hydroxyvitamin D₃ for the immunization and 25-hydroxyvitamin D₂ is also preferably coupled to the matrix at position 3.

The converse procedure is also successful i.e. immunization with a 25-hydroxyvitamin D₂ conjugate and immunosorption with a matrix to which 25-hydroxyvitamin D₃ is coupled. In another preferred element of the invention a 25-hydroxyvitamin D₂ conjugate is used as the immunogen conjugate and the antibodies generated with this immunogen are immuno-adsorbed onto a 25-hydroxyvitamin D₃ matrix.

EAH-Sepharose has proven to be particularly suitable as the matrix material for the immunosorption. In a preferred embodiment the antibodies contained in the serum or plasma from an immunization against 25-hydroxyvitamin D₃ or 25-hydroxy-vitamin D₂ are purified by immunosorption using a matrix which contains 25-hydroxyvitamin D₂ or 25-hydroxyvitamin D₃. EAH-Sepharose is a preferred column material.

Using the procedure previously described in detail i.e. for example immunization with a 25-hydroxyvitamin D₃ conjugate and immunosorption using a 25-hydroxyvitamin D₂ conjugate it is possible to reproducibly produce antibodies which react with both forms of 25-hydroxyvitamin D i.e. with 25-hydroxyvitamin D₂ and 25-hydroxyvitamin D₃. The antibodies obtained in this manner have a cross-reaction of the order of magnitude of 10 % to 1000 %. Thus in a preferred embodiment the present invention concerns for example antibodies against 25-hydroxyvitamin D₃ which have a cross-reaction of 10 % to 1000 % with 25-hydroxyvitamin D₂. The cross-reaction with the complementary 25-hydroxyvitamin D form is also preferably in a range of 20 % to 500 %. The extent of cross-reactions is determined in an immunological test method using the antibodies produced according to the present invention. An antibody produced against 25-hydroxyvitamin D₃ as a hapten for example has a cross-reaction of 10 % for 25-hydroxyvitamin D₂ if, when using the same analyte concentration of 25-hydroxyvitamin D₂ or 25-hydroxyvitamin D₃, only a tenth of 25-hydroxyvitamin D₃ is read-off on a calibration curve generated with 25-hydroxyvitamin D₃.

The antibodies against 25-hydroxyvitamin D produced by a process according to the invention have proven to be suitable for use in an automated test for 25-hydroxyvitamin D. Hence the present invention preferably concerns the use of an antibody against 25-hydroxyvitamin D in an immunological test for the detection of 25-hydroxyvitamin D. The test for 25-hydroxyvitamin D is preferably completely automated. The antibodies according to the invention are particularly preferably used in a test that can be carried out on automated Elecsys^{®} analyzers from Roche Diagnostics.

The teaching according to the present invention enables a person skilled in the art to put together a test kit which contains all components required for the detection of 25-hydroxyvitamin D. A preferred test kit for detecting 25-hydroxyvitamin D is in particular characterized in that such a kit contains an antibody against 25-hydroxy-vitamin D which recognizes both forms of 25-hydroxyvitamin D i.e. has a cross-reaction of 10 % to 1000 % to the complementary form of 25-hydroxyvitamin D in each case.

The test is preferably carried out as a competitive immunoassay in which the antibodies against 25-hydroxyvitamin D according to the invention are preferably used as a detection reagent. In such a competitive test a 25-hydroxyvitamin D "wall antigen" added in a defined amount to the test competes with the 25-hydroxyvitamin D from the sample for the binding sites of the detection antibody. The more 25-hydroxyvitamin D is present in the sample the smaller is the detection signal.

In addition it has proven to be advantageous that the form of 25-hydroxyvitamin D present as the wall antigen in the competitive test corresponds to the form that is used in the immunosorption. If one for example immunizes with an immunogen containing 25-hydroxyvitamin D₃, immunosorption is carried out on a 25-hydroxy-vitamin D₂ matrix and a 25-hydroxyvitamin D₂ derivative is preferably used in the test as the wall antigen. The wall antigen is preferably also modified at the same ring position as the immunogen and as the 25-hydroxyvitamin D used on the matrix for immunosorption.

In a further preferred embodiment the present invention concerns an immunological detection method for 25-hydroxyvitamin D in which a polyclonal antibody is used which was obtained by immunization with a 25-hydroxyvitamin D conjugate and immunosorption to the complementary 25-hydroxyvitamin D conjugate and wherein in a competitive test a derivative of the 25-hydroxyvitamin D complementary to the immunogen is used as the wall antigen.

The invention is further elucidated by the following examples and figures. The actual protective scope results from the claims attached to this invention.

### Description of the figures:

- **Figure 1:**: **Schematic representation of the synthesis of a 25-hydroxyvitamin D₃ immunogen**
Vitamin D₃ was activated via position 3 of the backbone from formula II and coupled to keyhole limpet hemocyanine (KLH) as the carrier.

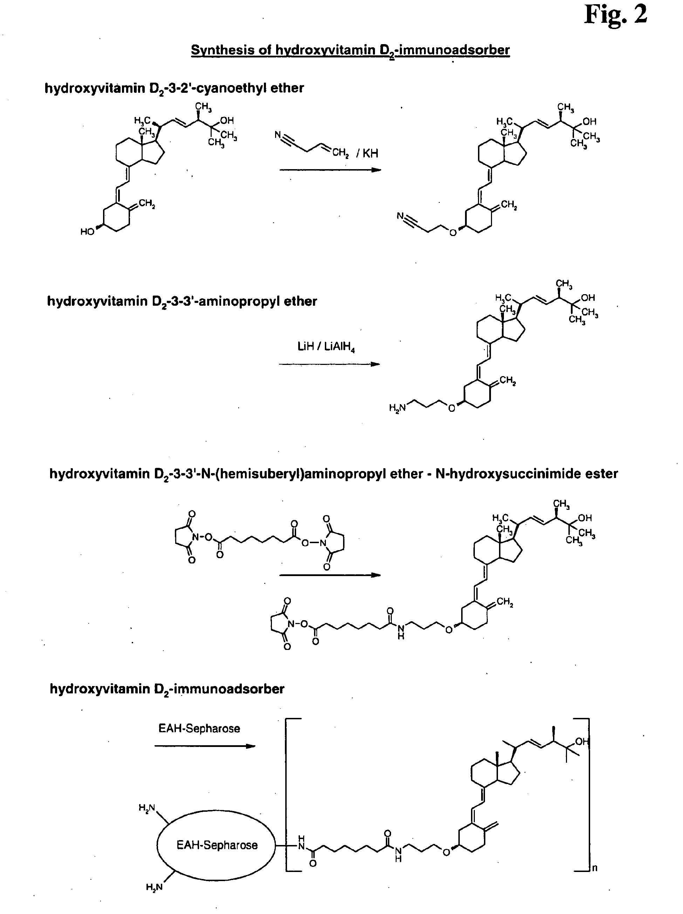

- **Figure 2:**: **Schematic representation of the synthesis of a 25-hydroxyvitamin D₂ immunoadsorber**
Vitamin D₂ was activated via position 3 of the backbone-from formula I and coupled to the matrix material EAH-Sepharose.

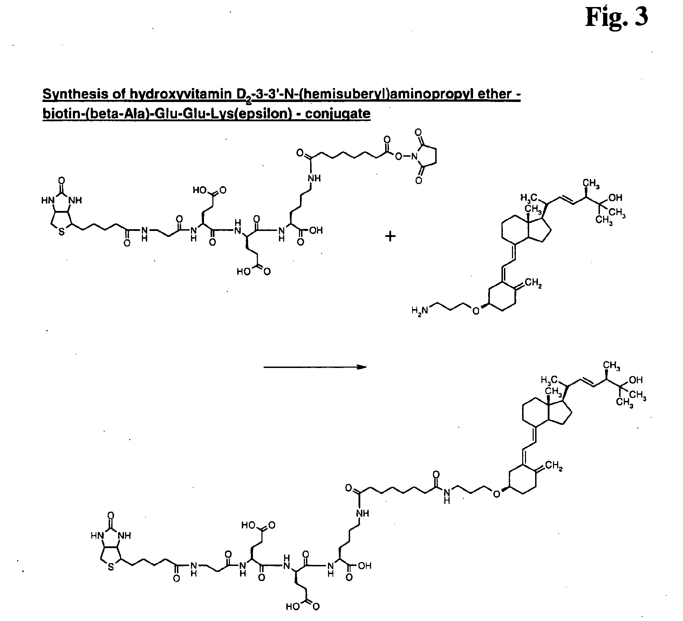

- **Figure 3:**: **Schematic representation of the synthesis of biotinylated vitamin D₂** The steps for synthesizing 25-hydroxyvitamin D₂ used as a wall antigen are shown diagrammatically.
- **Figure 4:**: **Immunoassay using antibodies of the prior art**
The content of 25-hydroxyvitamin D was determined in a total of 32 samples by means of an immunoassay as well as by means of HPLC.
The values determined in the immunoassay are plotted on the Y axis and the HPLC values on the X axis.
- **Figure 5:**: **Comparison of HPLC and LC-MS-MS**
The content of 25-hydroxyvitamin D was determined in a total of 66 samples by means of LC-MS-MS as well as by means of HPLC. The values determined in the LC-MS-MS are plotted on the Y axis and the HPLC values on the X axis.
- **Figure 6:**: **Comparison of an immunoassay using antibodies according to the invention and LC-MS-MS**
The content of 25-hydroxyvitamin D was determined in a total of 66 samples by means of an immunoassay based on antibodies according to the present invention as well as by means of HPLC. The values determined in the immunoassay are plotted on the Y axis and the HPLC values on the X axis.

### Example 1

### Synthesis of 25-hydroxyvitamin D₃-3-hemisuccinate-KLH

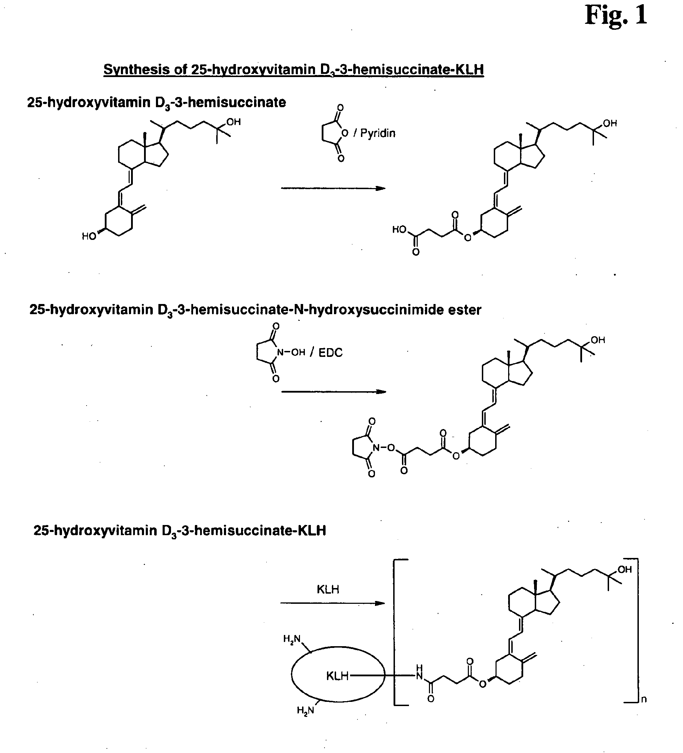

For this synthesis 25-hydroxyvitamin D₃ was chemically activated at position 3 (cf formula II) and coupled to KLH as an immunogen support. This synthesis via the intermediate steps 25-hydroxyvitamin D₃-3-hemisuccinate and 25-hydroxyvitamin D₃-3-hemisuccinate-N-hydroxysuccinimide ester is shown schematically in figure 1.

### 1.1 Preparation of 25-hydroxyvitamin D₃-3-hemisuccinate

10 mg (25 µmol) 25-hydroxyvitamin D₃ (Sigma-Aldrich, No. H-4014) is dissolved in 1 ml absolute pyridine and stirred for 4 days at room temperature in the dark with 125 mg (1.25 mmol) succinic anhydride. The reaction mixture is taken up in 10 ml ethyl acetate and in each case washed with 2 x 10 ml water, 0.1 M hydrochloric acid and subsequently again with water. The organic phase is dried using about 1 g anhydrous sodium sulfate, filtered and the solvent is removed in a vacuum. The residual solid is dried in a high vacuum. 10.5 mg (yield: 84 %) of a colourless solid is obtained.

### 1.2 Preparation of 25-hydroxyvitamin D₃-3-hemisuccinate-N-hydroxy-succinimide ester

10.0 mg (20 µmol) 25-hydroxyvitamin D₃-3-hemisuccinate is dissolved in 7 ml anhydrous dichloromethane and admixed with 2.76 mg (24 µmol) N-hydroxysuccinimide and 3.72 mg (24 µmol) N(3-dimethylaminopropyl)-N'-ethylcarbodiimide (EDC). It is stirred overnight under argon, the organic phase is then washed twice with 10 ml water, dried over about 1 g anhydrous sodium sulfate and filtered. The solvent is removed in a vacuum and the residual reaction product is dried for 3 h in a high vacuum. 11.3 mg (yield: 94 %) N-hydroxysuccinimide ester is obtained which is used for the conjugation without further purification.

### 1.3 Synthesis of 25-hydroxyvitamin D₃-3-hemisuccinate-KLH

150 mg keyhole limpet hemocyanin (KLH; Sigma-Aldrich No. H 8283) is dissolved in 25 ml 0.1 M potassium phosphate buffer, pH 8.0 and 11.3 mg of the N-hydroxysuccinimide ester in 2 ml DMSO is added. It is stirred overnight at room temperature, the product is subsequently purified by means of a gel column (AcA 202, column volume 0.51; 0.1 M potassium phosphate buffer pH 7.0). The fractions containing the conjugated protein are detected by means of UV absorption (λ = 256 nm) and pooled. 10 % Glycerol is added and the grey opalescent solution is used for the immunization.

### Example 2

### Production and isolation of antibodies against 25-hydroxyvitamin D₃

### 2.1 Immunization

The antibodies are produced in sheep. The 25-hydroxyvitamin D₃-3-hemisuccinate KLH conjugate from example 1 is used for the immunization. The immunization dosage is 0.1 mg per animal. The first immunization is carried out in complete Freud's adjuvant. Further immunizations take place at .4 week intervals in incomplete Freud's adjuvant over a period of 10 months. Serum is collected in the middle of each immunization interval.

### 2.2 Purification of the polyclonal sheep antibodies

The lipid-containing components are removed from the serum of the sheep immunized with 25-hydroxyvitamin D₃-3-hemisuccinate-KLH conjugate with the aid of Aerosil^{®} (1.5 %). Subsequently the immunoglobulins are precipitated with ammonium sulfate (1.7 M). The precipitate is dialysed against 15 mM potassium phosphate buffer containing 50 mM NaCl, pH 7.0 and subsequently purified chromatographically by DEAE sepharose. The IgG fraction (= PAB <25-hydroxyvitamin D₃> S-IgG (DE)) is obtained from the flow-through of this chromatography column.

### 2.3 Affinity chromatography to purify 25-hydroxyvitamin D-specific antibodies

An immunadsorber which contains conjugated 25-hydroxyvitamin D₂ as the specificity determinant is prepared for the immunochromatographic purification of the polyclonal antibodies. The immunadsorber is obtained by the following steps:

### a) Synthesis of hydroxyvitamin D₂-3-2'-cyanoethyl ether

20.6 mg (50 µmol) 25-hydroxyvitamin D₂ (Fluka No. 17937) is dissolved in a 25 ml three necked round bottom flask with an internal thermometer in 10 ml dry acetonitrile under an argon atmosphere. 1.5 ml tert.-butanol/acetonitrile (9:1) is added to the solution and cooled to 6°C in an ice bath. Subsequently 820 µl of an acrylonitrile solution (86 µl acrylonitrile in 1.0 ml acetonitrile) is added and stirred for 15 minutes at 6°C. Then 205 µl of a potassium hydride solution (25 mg KH in 0.5 ml tert.-butanol/acetonitrile 9:1) is added. A brief flocculation occurs after which a clear solution is obtained. The reaction solution is stirred for a further 45 minutes at 6°C and subsequently for 60 minutes at 4°C.

Subsequently the reaction solution is diluted with 10 ml methyl-tert.-butyl ether and washed twice with 10 ml H₂O each time. The organic phase is dried with about 1 g anhydrous sodium sulfate, filtered over a G3 glass frit and evaporated on a rotary evaporator. It is dried in a high vacuum to a viscous clear residue with a mass of about 55 mg.

### b) Synthesis of hydroxyvitamin D₂-3-3'-aminopropyl ether

The entire nitrile obtained above is dissolved in 15 ml diethyl ether and admixed with a suspension of 7.5 mg lithium hydride in 7.5 ml diethyl ether while stirring. The reaction mixture is stirred for 1 hour at room temperature. Afterwards a suspension of 38.4 lithium aluminium hydride in 6.6 ml diethyl ether is added. This results in a strong turbidity of the mixture. The reaction mixture is stirred for a further hour at room temperature, then the reaction mixture is cooled to 0-5°C in an ice bath and 35 ml water is carefully added. The pH is made strongly basic by addition of 6.6 ml 10 M potassium hydroxide solution.

It is extracted three times with 65 ml methyl-tert.-butyl ether each time. The combined organic phases are dried using about 5 g anhydrous sodium sulfate, filtered and evaporated at room temperature on a rotary evaporator. The residue is dried to mass constancy using an oil pump. The crude product is dissolved in 5 ml DMSO and 3.0 ml acetonitrile and purified by means of preparative HPLC.
eluant A = Millipore H2O + 0.1 % trifluoroacetic acid;
eluant B = 95 % acetonitrile + 5 % Millipore H2O + 0.1 % TFA;
gradient: from 50 % B to 100 % B in 100 min

| | |
|---|---|
| flow rate: | 30 ml/min |
| temperature: | room temperature |
| column dimension: | Ø = 5.0 cm; L = 25 cm; |
| column material: | Vydac C18/300Å/15-20 µm |
| det. wavelength: | 226 nm |

Fractions whose product content is higher than 85 % according to analytical HPLC (Vydac C18/300Å/5 µm; 4.6 x 250 mm) are pooled in a round bottom flask and lyophilized. 13.7 mg (yield: 58 %) of a colourless lyophilisate is obtained.

### c) Synthesis of hydroxyvitamin D₂-3-3'-N-(hemisuberyl)aminopropyl-ether-N-hydroxysuccinimide ester

11.7 mg (25 µmol) of the amino derivative is dissolved in 5 ml freshly distilled DMF and 92 mg (250 µmol) suberic acid-N-hydroxysuccinimide ester is added. 3.5 µl triethylamine is added and the solution is stirred overnight under argon. The crude product is purified by preparative HPLC (conditions as above). 10.1 mg (yield: 56 %) N-hydroxysuccinimide ester is obtained after lyophilization.

### d) Synthesis of the hydroxyvitamin D₂ immunoadsorber

20 ml EAH Sepharose (Amersham Biosciences, No. 17-0569-03) is washed with 200 ml 0.5 M sodium chloride solution on a G3 glass frit and equilibrated with 200 ml 0.03 M potassium phosphate buffer pH 7.1. After excess liquid has drained off through the frit, the suspension is taken up in 200 ml of the same buffer and 1.7 mg (2.3 µmol) of the N-hydroxysuccinimide ester in 10 ml DMSO is added. The reaction mixture is agitated overnight at room temperature on a shaker. It is again transferred to a G3 glass frit, allowed to drain and washed with 500 ml 0.05 M potassium phosphate buffer/0.15 M sodium chloride, pH 7.0. After complete drainage, it is resuspended in 25 ml of the same buffer and 0.15 ml of a 25 % sodium azide solution is added for preservation.

### e) Purification of the antibodies

10 ml of the affinity matrix from d) is packed into a column and equilibrated with a buffer consisting of 50 mM potassium phosphate, 150 mM NaCl at a pH of 7.5 (PBS). 3.6 g of PAB <25-hydroxyvitamin D₃> S-IgG (DE) is loaded onto the column. The column is washed stepwise with PBS, 0.5 M NaCl solution containing 0.05 % Tween^{®} 20 and 30 mM sodium chloride. The specifically bound immunoglobulin is detached from the affinity matrix with 3 mM HCl solution. The HCl eluate is dialysed against 1 mM ethyl acetate and subsequently lyophilized. The lyophilisate is dissolved in PBS, aggregates are removed by chromatography on Superdex 200^{®} and the immunoadsorbed polyclonal antibodies obtained in this manner are used in a further step. The immunoaffinity matrix is regenerated with 1 M propionic acid and preserved in a solution of PBS containing 0.9 % sodium azide.

### Example 3

### Assays for the detection of 25-hydroxyvitamin D

Commercial assays are used according to the manufacturer's instructions. The 25-hydroxyvitamin D determinations are carried out by means of HPLC (test for 25(OH)vitamin D₃, from the "Immundiagnostik" Company, Bensheim, order No. KC 3400) or by means of LC-MS-MS (Vogeser, M. et al., Clin. Chem. 50 (2004) 1415-1417) as described in the literature.

The preparation of the ingredients and the general test procedure for a new immunological test is described in the following on the basis of antibodies produced according to the invention:

### 3.1 Synthesis of hydroxyvitamin D₂-3-3'-N-(hemisuberyl)aminopropyl-ether-biotin-(beta-Ala)-Glu-Glu-Lys(epsilon) conjugate (= Ag-Bi)

13.7 mg (25 µmol) hydroxyvitamin D₂-3-3'-aminopropyl ether is dissolved in 3.5 ml DMSO, 28.7 mg (30 µmol) biotin-(beta-Ala)-Glu-Glu-Lys(epsilon)-hemisuberate-N-hydroxysuccinimide ester (Roche Applied Science, No. 11866656) and 12.5 µl triethylamine are added and it is stirred overnight at room temperature. The reaction solution is diluted with 4.5 ml DMSO, filtered through a 0.45 µm microfilter and subsequently purified by means of preparative HPLC (conditions see example 2.3 b)). Fractions that contain more than 85 % product according to analytical HPLC are pooled and lyophilized. 9.8 mg (yield: 30 %) purified biotin conjugate is obtained.

### 3.2 Ruthenylation of polyclonal antibodies against 25-hydroxyvitamin D (= PAB-Ru) purified by affinity chromatography

The affinity-purified antibodies according to example 2.3 e) are transferred to 100 mM potassium phosphate buffer, pH 8.5 and the protein concentration is adjusted to 1 mg/ml. The ruthenylation reagent (ruthenium (II) tris (bipyridyl)-N-hydroxysuccinimide ester) is dissolved in DMSO and added to the antibody solution at a molar ratio of 7.5 to 1. After a reaction time of 60 min the reaction is stopped by addition of 1-lysine and the excess labelling reagent is separated by gel permeation chromatography on Sephadex G25.

### 3.3 Test procedure in the immunoassay

The sample is measured using an Elecsys^{®} system from the Roche Diagnostics company. 25 µl sample is mixed with 30 µl release reagent and simultaneously or sequentially with 15 µl ruthenylated detection antibody and incubated for 9 minutes. In the next step the biotinylated wall antigen (50 µl) is added and the pH value is kept in the desired range by further addition of release reagent (50 µl). After a further 9 minutes incubation magnetizable polystyrene particles coated with streptavidin (SA) (30 µl) are added and after a further incubation for 9 minutes, the amount of bound ruthenylated antibody is determined as usual.

The solution containing the ruthenylated <25-OH-vitamin D> antibody conjugate contains:

| | |
|---|---|
| 20 mM | phosphate buffer, pH 6.5 |
| 0.1 % | oxypyrion |
| 0.1 % | MIT (N-methylisothiazolone-HCl) |
| 10 % | DMSO (dimethyl sulfoxide) |
| 1 % | EtOH (ethanol) |
| 0.1 % | polydocanol |
| 1 % | rabbit IgG (DET) |
| 2.0 µg/ml | PAB-Ru (from example 3.2) |

The release reagent contains:

| | |
|---|---|
| 220 mM | acetate buffer, pH 4.0 |
| 0.1 % | oxypyrion |
| 0.1 % | MIT |
| 10 % | DMSO |
| 1% | EtOH |
| 0.1 % | polydocanol |
| 0.2 % | rabbit IgG |

The solution with the biotinylated wall antigen contains:

| | |
|---|---|
| 20 mM | phosphate buffer, pH 6.5 |
| 0.1 % | oxypyrion |
| 10 % | DMSO |
| 1% | EtOH |
| 0.1% | polydocanol |
| 0.2 % | rabbit IgG |
| 0.18 µg/ml | Ag-Bi (from example 3.1) |

The suspension with SA-coated latex particles contains:

| | |
|---|---|
| 0.72 mg/ml | SA-coated magnetizable polystyrene particles having a binding capacity of 470 ng/ml. |

### Example 4

### Results and discussion

### 4.1 Antibodies which are obtained using 25-hydroxyvitamin D₃ as an immunogen as well as an immunoadsorber

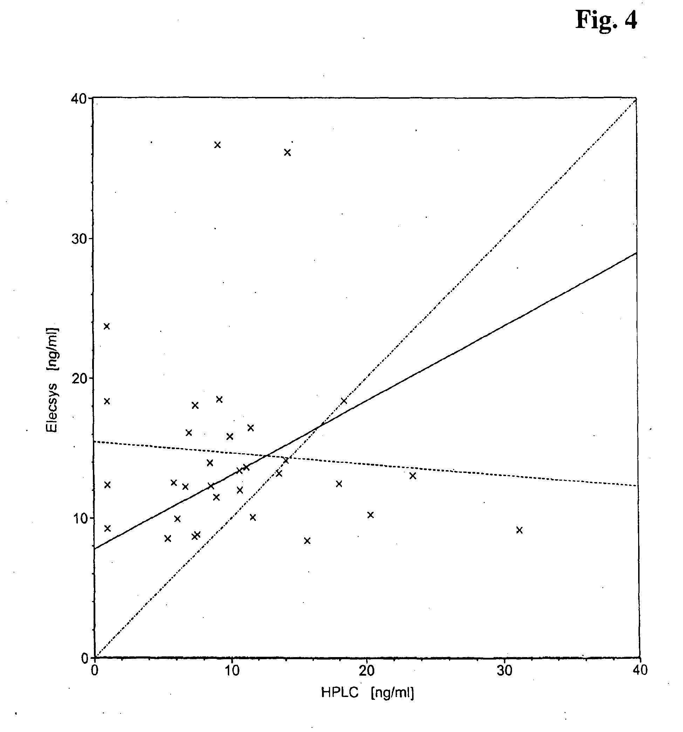

In many (unsuccessful) experiments antibodies were used which had been produced according to methods of the prior art i.e. immunization with and immunosorption to 25-hydroxyvitamin D₃. Figure 4 shows as an example that these antibodies are not suitable for reliably determining 25-hydroxyvitamin D. Figure 4 clearly shows that 25-hydroxyvitamin D values determined in an immunoassay using these antibodies do not correlate with the reference method (HPLC).

### 4.2 Comparison of HPLC with LC-MS-MS

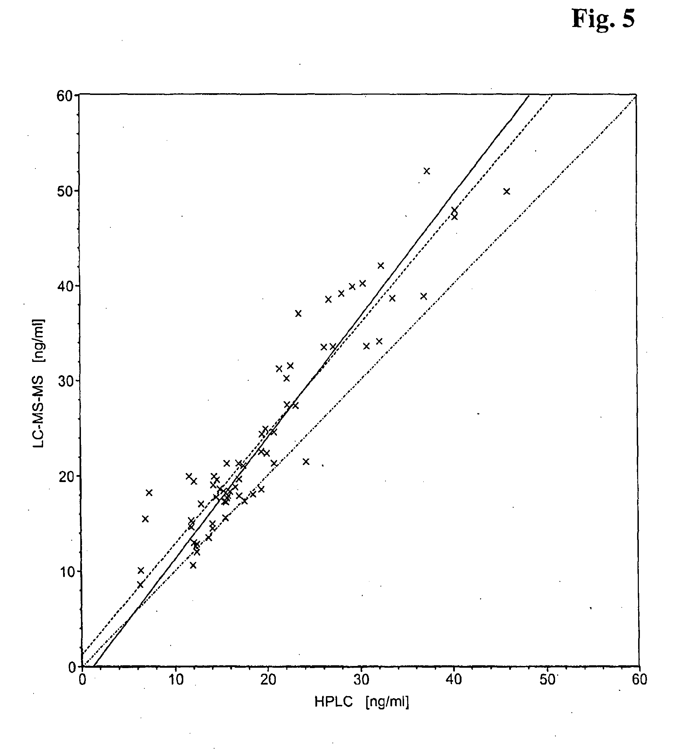

The detection of vitamin D metabolites by LC-MS-MS as described in Vogeser, M., et al., Clin. Chem. 50 (2004) 1415-1417 is increasingly becoming the reference method for vitamin D metabolite determinations. It was therefore investigated whether the previous HPLC reference method results in comparable values to the newer LC-MS-MS reference method. As can be seen from figure 5, both reference methods compare well. A correlation coefficient of 0.94 was determined by linear regression.

### 4.3 Immunoassay using the antibodies against 25-hydroxyvitamin D according to the invention

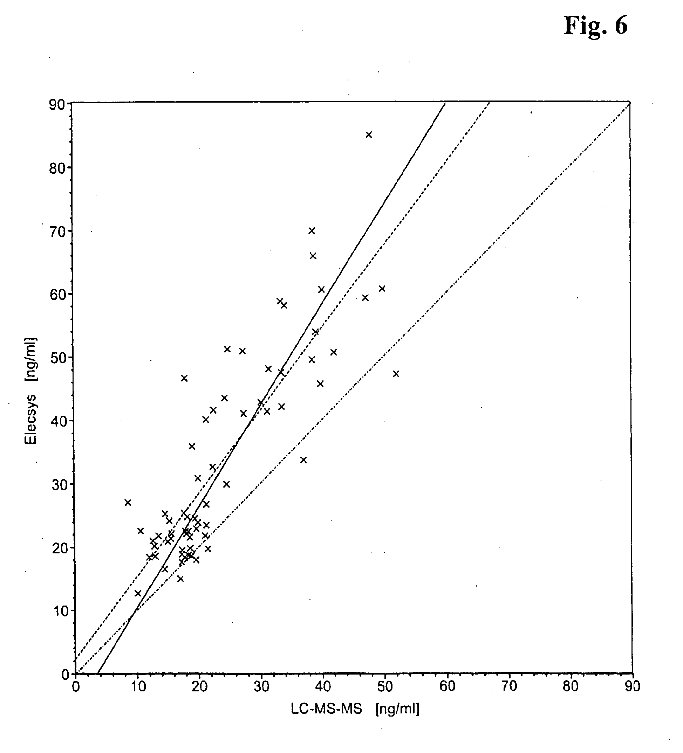

A total of 66 samples are compared in the new immunological test as well as by means of LC-MS-MS with regard to their content of 25-hydroxyvitamin D. As can be seen from figure 6 the values determined with both methods correlate very well. Linear regression yields a correlation coefficient of 0.85. This is surprisingly high considering that both analytical methods are based on completely different principles.

Thus a test for the detection of 25-hydroxyvitamin D can be established using the antibodies according , to the present invention, which enables a reliable determination of 25-hydroxyvitamin D.

## Claims

1. Process for the production of antibodies against 25-hydroxyvitamin D comprising the steps
a) immunizing an experimental animal with a conjugate which contains 25-hydroxyvitamin D₃ or 25-hydroxyvitamin D₂ as the hapten,
b) isolating serum or plasma from the said experimental animal and
c) purifying the antibodies contained in the serum or plasma by immunosorption to a complementary matrix, comprising 25-hydroxyvitamin D₂ or 25-hydroxyvitamin D₃, respectively.

2. Process according to claim 1, **characterized in that** the linkage in the 25-hydroxyvitamin D₃ conjugate is via position 3 of 25-hydroxyvitamin D₃.

3. Process according to claim 1, **characterized in that** the linkage in the 25-hydroxyvitamin D₂ conjugate is via position 3 of 25-hydroxyvitamin D₂.

4. Process according to claim 2, **characterized in that** 25-hydroxyvitamin D₂ is linked to the matrix used for immunosorption via position 3 of 25-hydroxyvitamin D₂.

5. Process according to claim 3, **characterized in that** 25-hydroxyvitamin D₃ is linked to the matrix used for immunosorption via position 3 of 25-hydroxyvitamin D₃.

6. Antibody against 25-hydroxyvitamin D₃ which has a cross-reaction as determined in a competitive immunoassay of 10 % to 1000 % with 25-hydroxyvitamin D₂.

7. Antibody against 25-hydroxyvitamin D₃ which has a cross-reaction as determined in a competitive immunoassay of 20 % to 500 % with 25-hydroxyvitamin D₂.

8. Use of an antibody according to claim 6 or 7 in a test for the detection of 25-hydroxyvitamin D₃.

9. Test kit for the detection of 25-hydroxyvitamin D₃, **characterized in that** this kit contains an antibody according to claim 6 or 7.

## Patentansprüche

1. Verfahren zur Herstellung von Antikörpern gegen 25-Hydroxyvitamin D, umfassend die folgenden Schritte:
a) Immunisieren eines Versuchstiers mit einem Konjugat, das 25-Hydroxyvitamin D₃ oder 25-Hydroxyvitamin D₂ als Hapten enthält,
b) Isolieren von Serum oder Plasma aus dem Versuchstier und
c) Aufreinigen der in dem Serum oder Plasma enthaltenen Antikörper durch Immunsorption an eine komplementäre Matrix umfassend 25-Hydroxyvitamin D₂ bzw. 25-Hydroxyvitamin D₃.

2. Verfahren nach Anspruch 1, **dadurch gekennzeichnet, daß** die Bindung in dem 25-Hydroxyvitamin-D₃-Konjugat über die Position 3 des 25-Hydroxyvitamin D₃ erfolgt.

3. Verfahren nach Anspruch 1, **dadurch gekennzeichnet, daß** die Bindung in dem 25-Hydroxyvitamin-D₂-Konjugat über die Position 3 des 25-Hydroxyvitamin D₂ erfolgt.

4. Verfahren nach Anspruch 2, **dadurch gekennzeichnet, daß** 25-Hydroxyvitamin D₂ an die für die Immunsorption verwendete Matrix über die Position 3 des 25-Hydroxyvitamin D₂ gebunden wird.

5. Verfahren nach Anspruch 3, **dadurch gekennzeichnet, daß** 25-Hydroxyvitamin D₃ an die für die Immunsorption verwendete Matrix über die Position 3 des 25-Hydroxyvitamin D₃ gebunden wird.

6. Antikörper gegen 25-Hydroxyvitamin D₃, das gemäß Bestimmung in einem kompetitiven Immunassay eine Kreuzreaktion mit 25-Hydroxyvitamin D₂ von 10% bis 1000% aufweist.

7. Antikörper gegen 25-Hydroxyvitamin D₃, das gemäß Bestimmung in einem kompetitiven Immunassay eine Kreuzreaktion mit 25-Hydroxyvitamin D₂ von 20% bis 500% aufweist.

8. Verwendung eines Antikörpers nach Anspruch 6 oder 7 in einem Test für den Nachweis von 25-Hydroxyvitamin D₃.

9. Testkit für den Nachweis von 25-Hydroxyvitamin D₃, **dadurch gekennzeichnet, daß** das Kit einen Antikörper nach Anspruch 6 oder 7 enthält.

## Revendications

1. Procédé pour la préparation d'anticorps dirigés contre la 25-hydroxyvitamine D, comprenant les étapes
a) d'immunisation d'un animal expérimental avec un conjugué qui contient de la 25-hydroxyvitamine D₃ ou de la 25-hydroxyvitamine D₂ à titre d'haptène ,
b) d'isolation du sérum ou du plasma dudit animal expérimental ; et
c) de purification des anticorps contenus dans le sérum ou dans le plasma par immunosorption à une matrice complémentaire, comprenant de la 25-hydroxyvitamine D₂ ou de la 25-hydroxyvitamine D₃, respectivement.

2. Procédé selon la revendication 1, **caractérisé en ce que** la liaison dans le conjugué de la 25-hydroxyvitamine D₃ a lieu via la position 3 de la 25-hydroxyvitamine D₃.

3. Procédé selon la revendication 1, **caractérisé en ce que** la liaison dans le conjugué de la 25-hydroxyvitamine D₂ a lieu via la position 3 de la 25-hydroxyvitamine D₂.

4. Procédé selon la revendication 2, **caractérisé en ce que** la 25-hydroxyvitamine D₂ est liée à la matrice que l'on utilise pour l'immunosorption via la position 3 de la 25-hydroxyvitamine D₂.

5. Procédé selon la revendication 3, **caractérisé en ce que** la 25-hydroxyvitamine D₃ est liée à la matrice que l'on utilise pour l'immunosorption via la position 3 de la 25-hydroxyvitamine D₃.

6. Anticorps dirigé contre la 25-hydroxyvitamine D₃, qui manifeste une réaction croisée comme déterminé dans un immunodosage compétitif, s'élevant de 10 % à 100 % avec la 25-hydroxyvitamine D₂.

7. Anticorps dirigé contre la 25-hydroxyvitamine D₃, qui manifeste une réaction croisée comme déterminé dans un immunodosage compétitif, s'élevant de 20 % à 500 % avec la 25-hydroxyvitamine D₂.

8. Utilisation d'un anticorps selon la revendication 6 ou 7 dans un test pour la détection de la 25-hydroxyvitamine D₃.

9. Kit pour la détection de la 25-hydroxyvitamine D₃, **caractérisé en ce que** ce nécessaire contient un anticorps selon la revendication 6 ou 7.
